Europäisches Patentamt

(19) **European Patent Office**

Office européen des brevets

(11) Veröffentlichungsnummer: **0 141 356**
**B1**

(12) **EUROPÄISCHE PATENTSCHRIFT**

(45) Veröffentlichungstag der Patentschrift:
27.08.86

(51) Int. Cl.⁴: **C 07 C 7/08** // B01D3/40

(21) Anmeldenummer: **84112672.5**

(22) Anmeldetag: **19.10.84**

(54) Verfahren zur Gewinnung eines konjugierten Diolefins und/oder Olefins aus einem C4- oder C5-Kohlenwasserstoffgemisch.

(30) Priorität: **28.10.83 DE 3339157**

(43) Veröffentlichungstag der Anmeldung:
**15.05.85 Patentblatt 85/20**

(45) Bekanntmachung des Hinweises auf die Patenterteilung:
**27.08.86 Patentblatt 86/35**

(84) Benannte Vertragsstaaten:
**AT BE DE FR GB IT NL SE**

(56) Entgegenhaltungen:
**DE - C - 1 568 902**

(73) Patentinhaber: **BASF Aktiengesellschaft,
Carl-Bosch-Strasse 38, D-6700 Ludwigshafen (DE)**

(72) Erfinder: **Lindner, Alfred, Dr., Ringstrasse 30,
D-6712 Bobenheim-Roxheim (DE)**
Erfinder: **Kaibel, Gerd, Robert-Bosch-Strasse 4,
D-6840 Lampertheim 1 (DE)**
Erfinder: **Wagner, Ulrich, Dr., Knospstrasse 7,
D-6703 Limburgerhof (DE)**
Erfinder: **Volkamer, Klaus, Dr., Heidelberger Ring 21,
D-6710 Frankenthal (DE)**
Erfinder: **Schneider, Klaus-Juergen, Dr.,
Triftbrunnenweg 16, D-6730 Neustadt (DE)**
Erfinder: **Weis, Josepha, Carl-Bosch-Ring 2,
D-6710 Frankenthal (DE)**
Erfinder: **Mayer, Hans-Horst, Friedrich-Ebert-Strasse 4,
D-6720 Speyer (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Gewinnung eines konjugierten Diolefins und/oder Olefins bzw. Olefingemisches aus einem das konjugierte Diolefin und/oder das Olefin bzw. Olefine enthaltenden $C_4$ - oder $C_5$ - Kohlenwasserstoffgemisch durch Extraktivdestillation mittels eines selektiven Lösungsmittels.

Die Extraktivdestillation ist ein bekanntes Verfahren zur Trennung von Gemischen, die durch übliche fraktionierte Destillation nicht leicht trennbar sind, z.B. wenn die zu trennenden Komponenten ein Azeotrop bilden oder geringe Unterschiede in den relativen Flüchtigkeiten besitzen. Bei der Extraktivdestillation wird in die Destillationskolonne eine solche Menge eines relativ schwer flüchtigen Lösungsmittels eingeführt, dass die Unterschiede in den relativen Flüchtigkeiten der zu trennenden Komponenten erhöht werden und damit eine destillative Trennung ermöglicht wird. Typische Anwendungsbeispiele für die extraktive Destillation finden sich beispielsweise in C.S. Robinson et al., „ Elements of Fractional Distillation", 4. Auflage, McGraw-Hill Book Company, Inc., New York (1959), S. 291.

Es ist bekannt, z.B. aus der DE-AS 15 68 902, DE-PS 11 63 795 oder aus The Soviet Chemical Industry, Nr. 11, November 1971, Seiten 719 bis 723, konjugierte Diolefine aus einem $C_4$ - oder $C_5$ - Kohlenwasserstoffgemisch durch Extraktivdestillation unter Verwendung eines selektiven Lösungsmittels oder Lösungsmittelgemisches zu gewinnen. Die selektiven Lösungsmittel können weitgehend wasserfrei verwendet werden. Dem selektiven Lösungsmittel kann auch zur Erhöhung der Selektivität und gegebenenfalls zur Erniedrigung des Siedepunktes Wasser zugemischt werden. Besonders bewährt hat sich für die Gewinnung eines konjugierten Diolefins aus einem $C_4$ - oder $C_5$ -Kohlenwasserstoffgemisch die Anwendung von zwei hintereinandergeschalteten Stufen der Extraktivdestillation.

In einer solchen zweistufigen Extraktivdestillation werden beispielsweise in der ersten Stufe der Extraktivdestillation ein die weniger löslichen Kohlenwasserstoffe enthaltendes Destillat (Raffinat) und ein das konjugierte Diolefin und die löslicheren Kohlenwasserstoffe und das selektive Lösungsmittel enthaltender Extrakt abgetrennt. Dieser Extrakt wird vom selektiven Lösungsmittel befreit, wobei ein Gemisch aus dem konjugierten Diolefin und den löslicheren Kohlenwasserstoffen erhalten wird. Dieses Gemisch wird einer zweiten Extraktivdestillation mit dem selektiven Lösungsmittel unterworfen, wobei das konjugierte Diolefin als Destillat und ein Extrakt erhalten werden, der die löslicheren Kohlenwasserstoffe und das selektive Lösungsmittel enthält. Der erhaltene Extrakt wird anschliessend vom selektiven Lösungsmittel befreit, wobei ein die löslicheren Kohlenwasserstoffe enthaltender Kohlenwasserstoffstrom erhalten wird. Dieses Verfahren ist jedoch nicht in jeder Beziehung zufriedenstellend.

Die vorliegende Erfindung soll nun eine Verbesserung der Arbeitsweise und Wirtschaftlichkeit der bekannten Verfahren bewirken.

Es wurde nun ein vorteilhaftes Verfahren gefunden, zur Gewinnung eines konjugierten Diolefins und/oder Olefins bzw. Olefingemischs aus einem das konjugierte Diolefin und/oder Olefin bzw. Olefin enthaltenden $C_4$ - oder $C_5$ - Kohlenwasserstoffgemisch, welches im selektiven Lösungsmittel schwerer als das konjugierte Diolefin und/oder Olefin bzw. die Olefine lösliche Kohlenwasserstoffe und im selektiven Lösungsmittel leichter lösliche Kohlenwasserstoffe als das konjugierte Diolefin und/oder Olefin bzw. die Olefine enthält, bei dem das $C_4$ - oder $C_5$ -Kohlenwasserstoffgemisch mittels Extraktivdestillation in ein die schwerer löslichen Kohlenwasserstoffe enthaltendes Destillat, einen das konjugierte Diolefin und/oder Olefin bzw. Olefingemisch enthaltenden Produktstrom und einen die leichter löslichen Kohlenwasserstoffe enthaltenden Strom aufgetrennt wird, welches dadurch gekennzeichnet ist, dass man die Extraktivdestillation unter Verwendung

— einer Destillatabzugszone (= Trennzone C),
— einer Zulaufzone für das Ausgangs-$C_4$ - oder $C_5$ -Kohlenwasserstoffgemisch (= Trennzone A),
— einer Produktabzugszone für den Produktstrom (= Trennzone B) und
— einer Ausstreifzone, in der das selektive Lösungsmittel zurückgewonnen und aus der der die leichter löslichen Kohlenwasserstoffe enthaltende Strom abgezogen wird (= Trennzone D) durchführt, wobei man

a) das Ausgangs-$C_4$ - oder $C_5$ -Kohlenwasserstoffgemisch der Trennzone A zuführt,

b) das der Extraktivdestillation zuzuführende selektive Lösungsmittel im oberen Teil der Trennzone C zugibt,

c) das im unteren Teil der Trennzone C beladene selektive Lösungsmittel zu einem Teil im oberen Teil der Trennzone A und zum anderen Teil im oberen Teil der Trennzone B zuführt,

d) das im unteren Teil der Trennzone A erhaltene beladene selektive Lösungsmittel und das im unteren Teil der Trennzone B erhaltene beladene selektive Lösungsmittel der Trennzone D zuführt, aus der der die leichter löslichen Kohlenwasserstoffe enthaltende Strom und ein Strom des von den Kohlenwasserstoffen ganz oder teilweise befreiten selektiven Lösungsmittels abgezogen werden,

e) aus der Trennzone B einen das konjugierte Diolefin und/oder Olefin bzw. Olefingemisch enthaltenden Produktstrom abzieht und

f) aus der Trennzone C das Destillat abzieht.

Nach dem neuen Verfahren kann die Umlaufmenge des selektiven Lösungsmittels unter sonst gleichen Bedingungen stark reduziert werden. Der Durchmesser der Trennkolonnen kann daher vermindert werden, so dass sich die Investitionskosten entsprechend verringern. Gleichzeitig kann auch der Dampf- und Strombedarf erheblich reduziert werden, so dass sich neben der Reduktion der Investitionskosten gleichzeitig eine erhebliche Reduktion der Energieverbräuche ergibt.

Als konjugierte Diolefine enthaltendes Kohlenwasserstoffgemisch, das als Ausgangsgemisch für das Verfahren der vorliegenden Erfindung geeignet ist, kann beispielsweise eine $C_4$- oder $C_5$-Fraktion, die durch thermisches Cracken einer Petroleumfraktion (z.B. LPG, Naphtha usw.) erhalten wurde, eine durch Dehydrierung von n-Butan und/oder n-Buten erhaltenes Butadien enthaltende Fraktion und eine durch Dehydrierung von Isopentan und/oder Isoamylen erhaltene Isopren enthaltende Fraktion verwendet werden. Im allgemeinen enthält das $C_4$-Kohlenwasserstoffgemisch 1,3-Butadien als konjugiertes Diolefin, Butane, n-Butene, Isobuten, Vinylacetylen, Ethylacetylen, 1,2-Butadien und gegebenenfalls geringe Mengen $C_3$- und/oder $C_5$-Kohlenwasserstoffe. Das $C_5$-Kohlenwasserstoffgemisch enthält in der Regel Isopren, trans- und cis-1,3-Pentadien, 1,4-Pentadien und Cyclopentadien als konjugierte Diolefine sowie Pentane, n-Pentene, Isoamylen, Cyclopentan, Cyclopenten, höhere Acetylene.

Diese konjugierte Diolefine enthaltenden Kohlenwasserstoffgemische können nach dem erfindungsgemässen Verfahren neben der Gewinnung des konjugierten Diolefins zur gleichzeitigen Gewinnung eines Olefins oder Olefingemisches verwendet werden. Mit besonderem Vorteil können aus den $C_4$- Kohlenwasserstoffgemischen neben 1,3-Butadien gleichzeitig n-Buten oder n-Butengemische gewonnen werden.

Geeignete selektive Lösungsmittel für das erfindungsgemässe Verfahren sind z.B. Butyrolacton, Nitrile wie Acetonitril, Propionitril, Methoxypropionitril, Ketone wie Aceton, Furfurol, N-alkylsubstituierte niedere aliphatische Säureamide, wie Dimethylformamid, Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon. Im allgemeinen werden N-alkylsubstituierte niedere aliphatische Säureamide oder N-alkylsubstituierte cyclische Säureamide verwendet. Mit besonderem Vorteil werden von den Lösungsmittel Dimethylformamid und insbesondere N-Methylpyrrolidon verwendet.

Es können jedoch auch Mischungen dieser Lösungsmittel untereinander, z.B. von N-Methylpyrrolidon mit Acetonitril, Mischungen dieser Lösungsmittel mit Wasser und/oder mit tert.-Butylethern, z.B. Methyl-tert.-butylether, Ethyl-tert.-butylether, Propyl-tert.-butylether, n- oder iso-Butyl-tert.-butylether, oder mit Tetrahydrofuran eingesetzt werden.

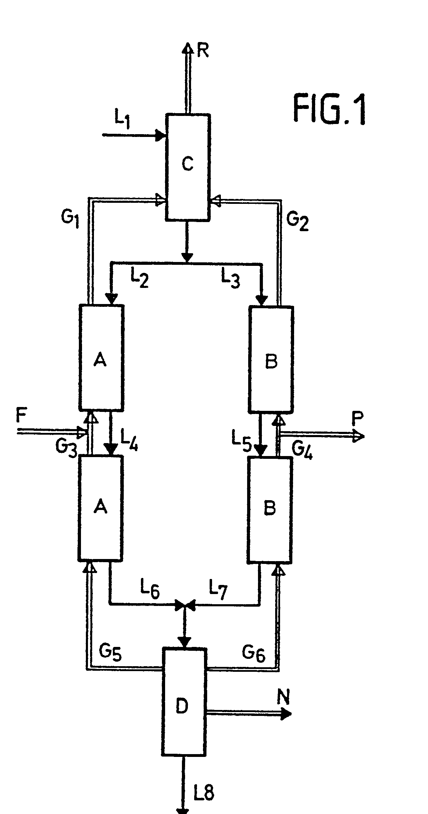

Fig. 1 ist ein schematisches Diagramm einer Ausführungsform der Extraktivdestillation gemäss dem erfindungsgemässen Verfahren. $L_1$ bis $L_8$ bedeuten die Führung des selektiven Lösungsmittels bzw. des mit Kohlenwasserstoffen beladenen selektiven Lösungsmittel und $G_1$ bis $G_6$ den Fluss der Kohlenwasserstoffe zwischen bzw. innerhalb der Trennzonen. F ist die Zuführung für das Ausgangs-Kohlenwasserstoffgemisch, R der Abzug für das Destillat, P der Abzug für den Produktstrom und N der Abzug für den die leichter löslichen Kohlenwasserstoffe enthaltenden Strom.

Gemäss dem erfindungsgemässen Verfahren wird das selektive Lösungsmittel im allgemeinen in der oberen Hälfte, vorzugsweise im oberen Drittel, zweckmässig an einem Punkt unterhalb des Abzuges des Destillat R, zugeführt. Das zugeführte Lösungsmittel kann mit Kohlenwasserstoffen vorbeladen, es kann jedoch auch, was bevorzugt wird, unbeladen sein bzw. nur eine geringe Restladung aufweisen. Vorzugsweise wird der Trennzone C das aus der Abstreifzone D erhaltene, ganz oder teilweise von Kohlenwasserstoffen befreite selektive Lösungsmittel zugeführt. Das im unteren Teil, im allgemeinen in der unteren Hälfte, vorzugsweise im unteren Drittel, beispielsweise am Boden von Trennzone C, abgezogene beladene selektive Lösungsmittel wird zu einem Teil (über $L_2$) im oberen Teil, im allgemeinen in der oberen Hälfte, vorzugsweise im oberen Drittel, insbesondere im oberen Viertel, der Trennzone A und zum anderen Teil (über $L_3$) im oberen Teil, im allgemeinen in der oberen Hälfte, vorzugsweise im oberen Drittel, insbesondere im oberen Viertel, der Trennzone B, vorzugsweise oberhalb des Zulaufpunktes des Ausgangs-$C_4$- oder $C_5$-Kohlenwasserstoffgemisches zur Trennzone A bzw. oberhalb des Abzugspunktes des Produktstroms aus Trennzone B zugeführt. Das Verhältnis, in dem das aus der Trennzone C abgezogene beladene Lösungsmittel auf die Trennzone A und B verteilt wird, beträgt im allgemeinen 1:50 bis 50:1, vorzugsweise 1:10 bis 10:1, insbesondere 5:1 bis 1:5.

Die in den Trennzonen A und B aufsteigenden, im Gegenstrom zum selektiven Lösungsmittel geführten Kohlenwasserstoffe, die im oberen Teil der Trennzonen A und B über $G_1$ und $G_2$ abgezogen werden, werden zweckmässig der Trennzone C im unteren Teil, im allgemeinen in der unteren Hälfte, vorzugsweise im unteren Drittel, insbesondere im unteren Viertel, zugeführt. Die Trennstufenzahl in den Trennzonen A und B kann gleich oder unterschiedlich sein. Das zu trennende Ausgangs-$C_4$- oder $C_5$-Kohlenwasserstoffgemisch wird der Trennzone A (über F) zugeführt, zweckmässig in einem mittleren Bereich, vorzugsweise in einem Bereich, der sich zwischen dem oberen Fünftel und dem unteren Fünftel, insbesondere zwischen dem oberen Viertel und dem unteren Viertel von Trennzone A erstreckt. Das Gemisch kann gasförmig oder flüssig zugeführt werden, wobei ein gasförmiger Zulauf bevorzugt ist. Es kann zweckmässig sein, einen oder mehrere weitere Kohlenwasserstoffzuläufe den Trennzonen A, B, und C und/oder D zuzuführen. Im allgemeinen handelt es sich bei den weiteren Kohlenwasserstoffzuläufen um aufzutrennende Kohlenwasserstoffgemische, die sich vom Ausgangs-$C_4$- oder $C_5$-Kohlenwasserstoffgemisch in der Zusammensetzung unterscheiden und für die in der Regel eine andere Zulaufstelle als die für das Ausgangs-$C_4$- oder $C_5$-Kohlenwasserstoffgemisch gewählt wird. Im allgemeinen wird für diese weiteren Kohlenwasserstoffzuläufe ein Zulaufpunkt in der Trennzone A bevorzugt, falls das weitere Kohlenwasserstoffgemisch sowohl leichter als auch schwerer als das Produkt lösliche Kohlenwasserstoffe in grösseren Mengen enthält. Für weitere Kohlenwasserstoffzuläufe, die ausser dem Produkt-Kohlenwasserstoff im wesentlichen nur schwerer als der Produkt-Kohlenwasserstoff lösliche Kohlenwasserstoffe enthalten, wird in der Regel ein Zulaufpunkt in der Trennzone B oberhalb des Produktabzugs, in der Trennzone C oder auch in der Trennzone A verwendet. Für weitere Kohlenwasserstoffzuläufe, die ausser dem Produkt-Kohlenwasserstoff im wesentlichen nur leichter als der Produkt-Kohlenwasserstoff lösliche Kohlenwasserstoffe enthalten, wird in der Regel ein Zulaufpunkt in der Trennzone B unterhalb des Produktabzuges, in der Trennzone A oder in der Trennzone D gewähltl. Es kann jedoch auch zweckmässig sein, für die weiteren Kohlenwasserstoffzuläufe andere als die beschriebenen bevorzugten Zulaufstellen zu wählen, z.B. um eine Ausstreifung zu verbessern, oder wenn eine destillative Abtrennung von den aus diesem Zulauf ins Produkt gelangenden Nebenkomponenten vorgesehen und möglich ist, oder wenn diese ins Produkt gelangende Nebenkomponente bei der Verarbeitung nicht stört.

Der das konjugierte Diolefin und/oder Olefin bzw. Olefingemisch enthaltende Produktstrom wird aus Trennzone B (über P) abgezogen, zweckmässig aus einem mittleren Bereich, vorzugsweise aus einem Bereich, der sich zwischen dem oberen Fünftel und dem unteren Fünftel, insbesondere zwischen dem oberen Viertel und dem unteren Viertel, von Trennzone B erstreckt. Er kann gasförmig oder flüssig, zweckmässig gelöst im Lösungsmittel, abgezogen werden, wobei ein gasförmiger Abzug bevorzugt ist. Es kann zweckmässig sein, aus den Trennzonen A, B, C und/oder D einen oder mehrere weitere Produktströme abzuziehen. Im allgemeinen handelt es sich bei den weiteren Produktströmen um Kohlenwasserstoffgemische, die sich vom ersten Produktstrom in der Zusammensetzung unterscheiden und deren Abzugsstelle von der Abzugsstelle des ersten Produktstromes verschieden ist. So kann es beispielsweise bei der Gewinnung von 1,3-Butadien aus einem $C_4$-Kohlenwasserstoffgemisch vorteilhaft sein, aus Trennstufe B neben dem 1,3-Butadien-Produkt als weiteres Produkt Butan-, 1,3-Butadien- und Acetylen-freies oder -armes 2-Buten-cis oder Buten-Gemisch abzuziehen. Solche Buten-Gemische können z.B. zur Gewinnung der reinen Butene mit Vorteil eingesetzt werden.

Das im unteren Teil, im allgemeinen in der unteren Hälfte, vorzugsweise im unteren Drittel, beispielsweise am Boden der Trennzone A abgezogene beladene selektive Lösungsmittel und das im unteren Teil, im allgemeinen in der unteren Hälfte, vorzugsweise im unteren Drittel, beispielsweise am Boden der Trennzone B abgezogene beladene selektive Lösungsmittel werden der Trennzone D (über $L_6$ und $L_7$) zugeführt, in der das die leichter löslichen Kohlenwasserstoffe enthaltende selektive Lösungsmittel von den Kohlenwasserstoffen ganz oder teilweise befreit wird. Die aus den Trennzonen A und B abgezogenen Ströme von beladenem Lösungsmittel können der Trennzone D nach vorheriger Vereinigung an einer Stelle zugeführt werden, oder sie können der Trennzone D getrennt an unterschiedlichen Stellen zugeführt werden. Die Zuführung der Lösungsmittelströme erfolgt zweckmässig im oberen Teil der Trennzone D, vorzugsweise in der oberen Hälfte, insbesondere im oberen Drittel, beispielsweise am Kopf der Trennzone D. Das ganz oder teilweise von den Kohlenwasserstoffen befreite selektive Lösungsmittel wird zweckmässig im unteren Teil der Trennzone D, vorzugsweise in der unteren Hälfte, insbesondere im unteren Drittel, beispielsweise am Boden der Trennzone D, abgezogen. Das abgezogene selektive Lösungsmittel wird zweckmässig wieder zur Trennzone C zurückgeführt. Das Ausstreifen bzw. Ausgasen der Kohlenwasserstoffe aus dem beladenen Lösungsmittel in der Trennzone D erfolgt im allgemeine durch Wärmezufuhr, beispielsweise über einen Aufkocher, zweckmässig im unteren Teil, vorzugsweise im unteren Drittel, insbesondere am Sumpf, von Trennzone D. Dabei werden neben der Befreiung des Lösungsmittels von den Kohlenwasserstoffen zumindest teilweise die aufsteigenden Kohlenwasserstoffdämpfe erzeugt, die in den Trennzonen für die Trennung der Kohlenwasserstoffe benötigt werden. Gleichzeitig wird aus der Trennzone D der die leichter löslichen Kohlenwasserstoffe enthaltende Strom abgezogen. Dieser Strom wird im allgemeinen an einer Stelle abgezogen, die oberhalb des Abzuges des ganz oder teilweise von Kohlenwasserstoffen befreiten Lösungsmitels und unterhalb der Zuführung des beladenen Lösungsmittels zur Trennzone D liegt, im allgemeinen in einem mittleren Bereich, der sich zwischen dem unteren und oberen Fünftel, vorzugsweise zwischen dem unteren und oberen Viertel der Trennzone D erstreckt. Es ist möglich, innerhalb der Trennzone D eine oder mehrere Entspannungen und Kompressionen vorzunehmen.

Die aus dem oberen Teil der Trennzone D, im allgemeinen aus der oberen Hälfte, vorzugsweise aus dem oberen Drittel, insbesondere aus dem oberen Viertel, beispielsweise am Kopf, erhaltenen Kohlenwasserstoffe werden zweckmässig auf die Trennzonen A und B verteilt, im allgemeinen im Verhältnis 1:50 bis 50:1, vorzugsweise 1:10 bis 10:1, insbesondere 1:5 bis 5:1. Die Kohlenwasserstoffe werden dabei den Trennzonen A und B zweckmässig im unteren Teil, vorzugsweise in der unteren Hälfte, insbesondere im unteren Drittel, beispielsweise am Boden, zugeführt. Bei der Verteilung der aus dem oberen Teil der Trennzone D erhaltenen Kohlenwasserstoffe auf die Trennzonen A und B ist es praktisch unerheblich, ob die Kohlenwasserstoffe gemeinsam aus der Trennzone D entnommen und dann aufgeteilt oder gleich als zwei getrennte Ströme entnommen werden. Entsprechendes gilt für die Verteilung des aus der Trennzone C abgezogenen Stromes von beladenem Lösungsmittel auf die Trennzonen A und B.

Die aus den oberen Teilen der Trennzonen A und B, im allgemeinen aus den oberen Hälften, vorzugsweise aus den oberen Dritteln, insbesondere oberen Vierteln, z.B. aus dem Kopf, erhaltenen Kohlenwasserstoffe werden zweckmässig ganz oder teilweise dem unteren Teil der Trennzone C, im allgemeinen der unteren Hälfte, vorzugsweise dem unteren Drittel, insbesondere dem unteren Viertel, zugeführt. Dabei können die Kohlenwasserstoffe der Trennzone C nach vorheriger Vereinigung an einer Stelle zugeführt werden, oder sie können der Trennzone D getrennt an unterschiedlichen Stellen zugeführt werden.

Zusätzlich zu der vorstehend beschriebenen Aufheizung am Sumpf der Trennzone D können weitere Wärmezufuhren in den Trennzonen A, B, C und/oder D vorgenommen werden. Bevorzugt werden für diese zusätzlichen Wärmezufuhren Stellen unterhalb des Zulaufs der Kohlenwasserstoffe bzw. des Produktabzugs. Solche Wärmezufuhren können z.B. durch Wärmeverbund mit dem ausgegasten Lösungsmittel erfolgen.

Aus den Trennzonen A, B, C und/oder D kann ebenfalls Wärmeabfuhr erfolgen. Bevorzugt wird eine Wärmeabfuhr aus der Trennzone A oberhalb des Zulaufs des Ausgangs-Kohlenwasserstoffgemisches sowie aus der Trennzone B oder C. Es kann jedoch auch erforderlich sein, Wärme aus der Trennzone D abzuführen, z.B. zur Einhaltung einer Maximaltemperatur eines Kompressors.

Die Trennzonen A bis D können konstruktiv in unterschiedliche Kolonnen aufgeteilt werden. Es können jedoch auch Teile von Trennzonen bzw. eine oder mehrere Trennzonen baulich miteinander kombiniert werden.

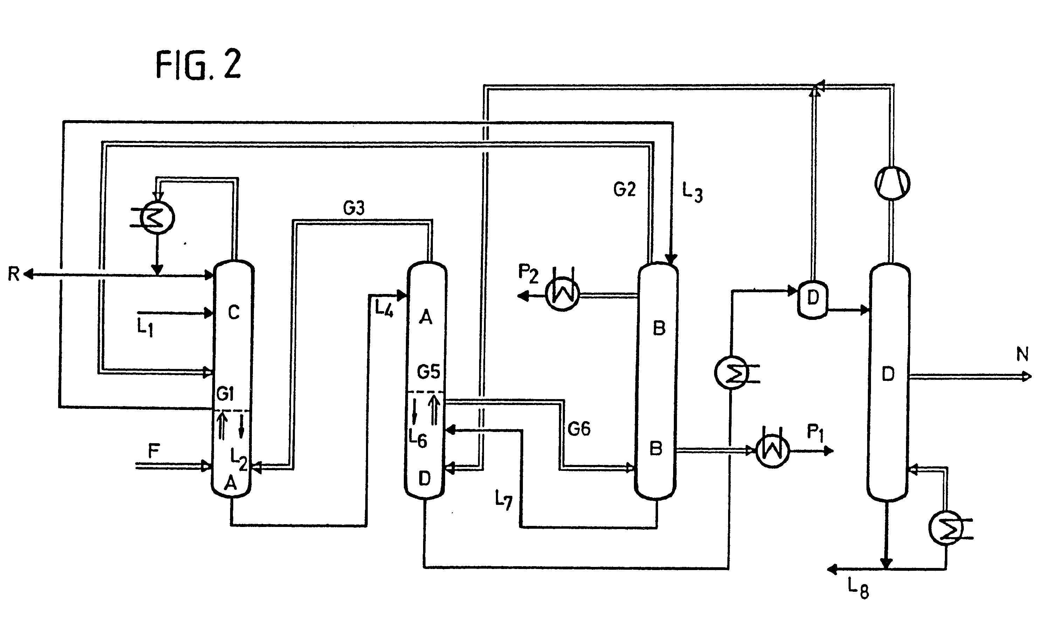

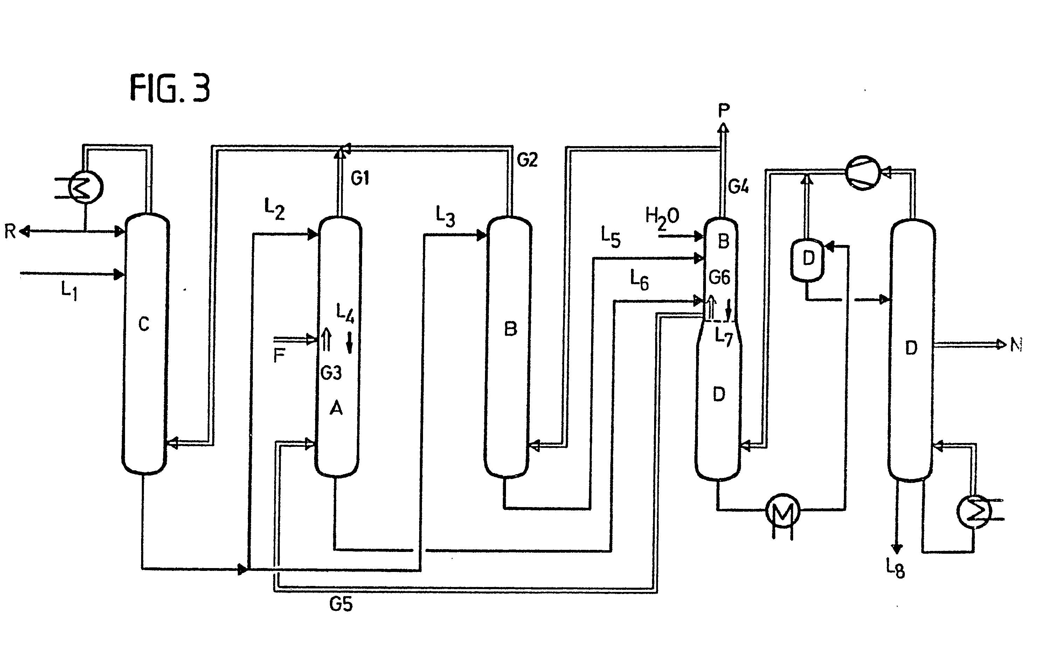

Figur 2 veranschaulicht beispielhaft eine der möglichen konstruktiven Lösungen. Zum leichteren Verständnis sind die Bezeichnungen der Ströme und Trennzonen ebenso wie in den nachfolgenden Figuren 3 bis 6 aus Figur 1 übernommen.

Trennzone C und Trennzone A oberhalb des Zulaufs F sind zu einer Kolonne kombiniert, ebenso die Trennzone A unterhalb des Zulaufs F und ein Teil der Trennzone D. Trennzone B ist als eine Kolonne ausgebildet. Die Trennzone D ist auf zwei Kolonnen und einen Gasabscheider verteilt.

Am Kopf der Trennzone C ist eine destillativ wirkende Trennzone aufgesetzt, um das Lösungsmittel und gegebenenfalls andere hochsiedende Komponenten zurückzuhalten. Ähnliche Trennzonen sind auch zur Reinigung des bzw. der Produkte P und des Nebenprodukts N, das die leichter löslichen Kohlenwasserstoffe enthält, möglich. Es können jedoch auch gas/flüssig- oder flüssig/flüssig-Wasserwäschen, partielle Kondensatoren oder Destillationen zu diesem Zweck eingesetzt werden.

Figur 2 enthält einen weiteren Abzug ($P_2$) aus der Trennzone B. Aus diesem Abzug $P_2$ wird beispielsweise bei der Gewinnung von 1,3-Butadien aus einem $C_4$-Kohlenwasserstoffgemisch zusätzlich zu dem über Leitung $P_1$ abgezogenen 1,3-Butadine-Produkt als weiteres Produkt 2-Buten-cis oder Buten-Gemisch abgezogen.

Figur 3 zeigt eine weitere Ausführungsform des erfindungsgemässen Verfahrens.

Sie dient zur Illustration, dass das Produkt P nicht notwendigerweise als Seitenabzug einer Kolonne abgezogen werden muss, sondern als Seitenabzug aus Trennzone B, die in diesem Beispiel auf zwei Kolonnen verteilt ist, abgezogen werden kann.

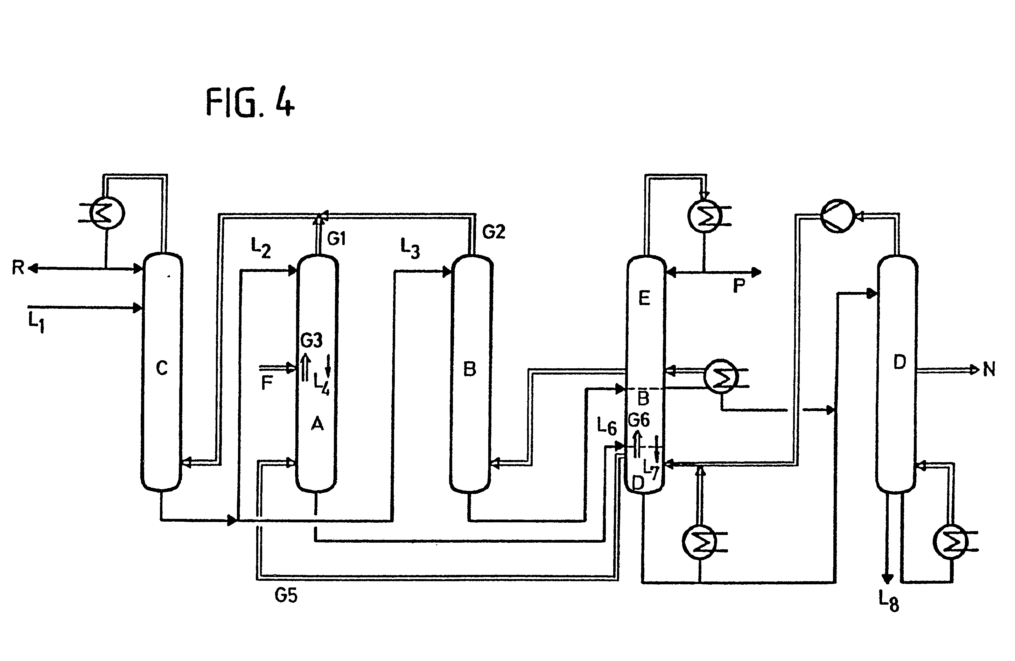

Figur 4 zeigt eine weitere Ausführungsform des erfindungsgemässen Verfahrens. Statt mit Wasserwäsche oder einer Kohlenwasserstoffwäsche, die nur zur Rückhaltung des Lösungsmittels dienen, wird eine destillative Trennzone E zur Feinreinigung des Produkts angewandt. Bei der Anwendung des erfindungsgemässen Verfahrens auf die Gewinnung von 1,3-Butadien aus einem $C_4$-Kohlenwasserstoffgemisch ergeben sich durch diese Arbeitsweise folgende Vorteile:

1) Sowohl leichter als 1,3-Butadien lösliche Komponenten, wie 1,2-Butadien und/oder 1-Butin, als auch schwerer als 1,3-Butadien lösliche Komponenten, wie die 2-Butene, werden durch Trennzone E zurückgehalten. In Trennzone B können deren Konzentrationen somit höher sein als ohne destillative Trennzone E. Da diese Komponenten Schlüsselkomponenten der Trennung sind, kann der Lösungsmittelumlauf reduziert und hochwertige Energie eingespart werden. Der Waschteil der Extraktivdestillation wirkt als Abtriebsteil der Destillation bezüglich 2-Buten, der Strippteil der Extraktivdestillation als Abtriebsteil der Destillation bezüglich 1,2-Butadien. Es ist möglich, den gesamten oder nur einen Teil des in Trennzone B aufsteigenden Kohlenwasserstoffdampfes abzuziehen und in Trennzone E einzuspeisen. Der im Oberteil der Trennzone B benötigte Kohlenwasserstoffdampf kann durch Rückführung aus Trennzone E und/oder durch Aufkochung des Lösungsmittels gewonnen werden. Es kann auch zweckmässig sein, vom unteren Teil der Trennzone E einen Teilstrom zu entnehmen und in Trennzone D und/oder A unterhalb des Zulaufs einzuspeisen, um Konzentrationsbäuche zu vermindern.

2) Die destillative Trennzone E kann mit preiswerter Abwärme, z.B. mit heissem Kondensat oder auf niedrigen Temperaturen befindlichem Dampf, betrieben werden, da das Temperaturniveau der Verdampfung niedrig liegt.

3) Trennzone E dient gleichzeitig zum Zurückhalten des selektiven Lösungsmittels.

Auch für die Anwendung der destillativen Trennzone E ist die örtliche Aufstellung, d.h. ob sie beispielsweise auf der Trennzone B aufgesetzt oder daneben aufgestellt wird, unerheblich.

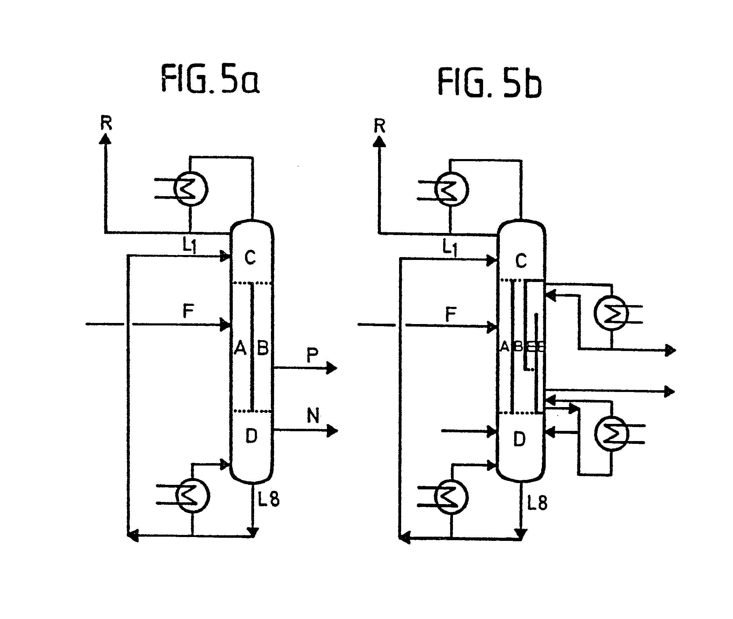

Weitere beispielshafte Ausführungsformen enthalten Figur 5a und Figur 5b. Die parallelen Trennzonen A und B werden apparativ in ein und demselben Kolonnenmantel untergebracht. Zur Trennung der beiden Zonen A und B voneinander ist ein senkrechtes Trennblech eingebaut. Gegebenenfalls können auch noch weitere Trennzonen, z.B. Destillationen, in den Kolonnenmantel mit eingebaut werden, die ebenfalls durch Trennblech von den übrigen Systemen abgetrennt werden. Beispielsweise zeigt Figur 5b eine destillative Trennzone E, die zusätzlich zu den extraktivdestillativen Trennzonen A und B in den Kolonnenmantel mit eingebaut ist. Dabei enthält Trennzone E selbst wiederum zwei teilweise parallele Trennzonen.

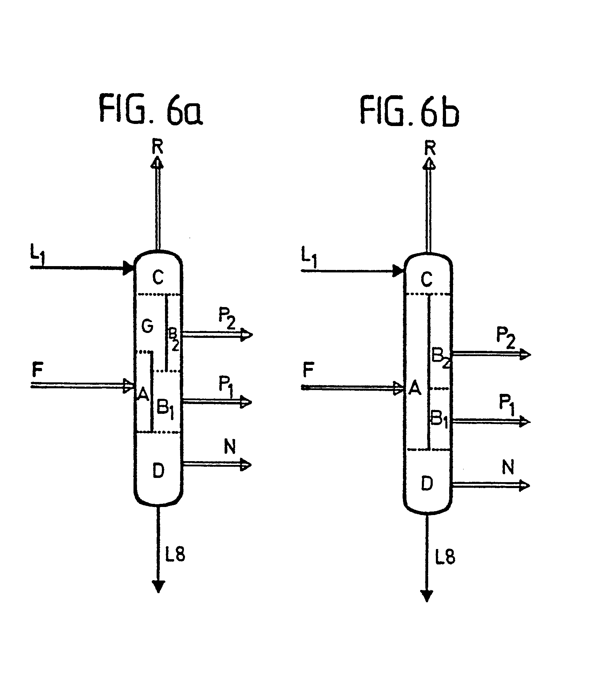

Figuren 6a und 6b zeigen beispielhafte Ausführungsformen für die gleichzeitige Gewinnung eines weiteren Produktes aus dem Ausgangs-$C_4$- oder $C_5$-Kohlenwasserstoffgemisch, z.B. aus einem $C_4$-Kohlenwasserstoffgemisch, neben der Gewinnung von 1,3-Butadien als Produkt $P_1$ die Gewinnung von Butenen als Produkt $P_2$. Die Trennzone B ist in zwei Teiltrennzonen $B_1$ und $B_2$ aufgeteilt, aus denen die Produkte $P_1$ (z.B. 1,3-Butadien) und $P_2$ (z.B. ein Butengemisch) abgezogen werden. Ausser den Trennzonen A, B, C und D ist in Figur 6a eine weitere Trennzone G eingeführt, in der das Destillat R nach oben angereichert und Produkte $P_1$ und $P_2$ nach unten angereichert werden.

Das nachstehende Beispiel dient der weiteren Erläuterung der Erfindung.

*Beispiel*

In einer Versuchsanlage zur Gewinnung von 1,3-Butadien mittels Extraktivdestillation wurde ein $C_4$-Kohlenwasserstoffgemisch gemäss dem Fliessbild von Figur 3 unter Verwendung von wässerigem N-Methylpyrrolidon (NMP) mit einem Gehalt von 8 Gew.% Wasser, bezogen auf das NMP/Wasser-Gemisch, als selektivem Lösungsmittel in ein 1,3-Butadien-Produkt, ein Butan/Buten-Gemisch und ein die $C_4$-Acetylene enthaltendes Gemisch aufgetrennt. Das eingesetzte $C_4$-Kohlenwasserstoffgemisch hatte die folgende Zusammensetzung:

| | |
|---|---|
| Butane | 4,1 Gew.% |
| Butene | 49,3 Gew.% |
| 1,3-Butadien | 45,0 Gew.% |
| $C_4$-Acetylene | 0,9 Gew.% |
| Andere Kohlenwasserstoffe | 0,7 Gew.% |

Der Versuchsanlage wurden 0,139 kg/h $C_4$-Kohlenwasserstoffgemisch über Leitung F zugeführt. Über Leitung P wurde 1,3-Butadien-Produkt mit einer Reinheit von 99,1% abgezogen. Das Butan/Buten-Gemisch wurde als Destillat über Leitung R erhalten, und das die $C_4$-Acetylene enthaltende Gemisch über Leitung N abgezogen. Über Leitung $L_1$ wurde der Versuchsanlage selektives Lösungsmittel in einer Menge von 1,46 kg/h zugeführt. Das zugeführte selektive Lösungsmittel wurde im Verhältnis 2,7:1 auf die Trennzonen A und B verteilt.

Führte man dagegen die Auftrennung des $C_4$-Kohlenwasserstoffgemisches in 1,3-Butadienprodukt mit einer Reinheit von 99,1%, Butan/Buten-Gemisch und das die $C_4$-Acetylene enthaltende Gemisch in einer konventionell verschalteten zweistufigen Extraktivdestillation durch, so mussten der ersten und zweiten Stufe der Extraktivdestillation insgesamt 1,66 kg/h des selektiven Lösungsmittels zugeführt werden, um den gleichen Trenneffekt zu erzielen, d.h. die beim erfindungsgemässen Verfahren zur Erzielung der gleichen Reinheit benötigte Lösungsmittelmenge war um 12% niedriger als die bei konventioneller Schaltung benötigte Gesamtlösungsmittelmenge. Mit dem neuen Verfahren konnten somit erhebliche Energieeinsparungen gegenüber dem konventionellen Verfahren erzielt werden.

## Patentansprüche

1. Verfahren zur Gewinnung eines konjugierten Diolefins und/oder Olefins bzw. Olefingemischs aus einem das konjugierte Diolefin und/oder Olefin bzw. Olefine enthaltenden $C_4$- oder $C_5$-Kohlenwasserstoffgemisch, welches im selektiven Lösungsmittel schwerer als das konjugierte Diolefin und/oder Olefin bzw. die Olefine lösliche Kohlenwasserstoffe und im selektiven Lösungsmittel leichter lösliche Kohlenwasserstoffe als das konjugierte Diolefin und/oder Olefin bzw. die Olefine enthält, bei dem das $C_4$- oder $C_5$-Kohlenwasserstoffgemisch mittels Extraktivdestillation in ein die schwerer löslichen Kohlenwasserstoffe enthaltendes Destillat, einen das konjugierte Diolefin und/oder Olefin bzw. Olefingemisch enthaltenden Produktstrom und einen die leichter löslichen Kohlenwasserstoffe enthaltenden Strom aufgetrennt wird, dadurch gekennzeichnet, dass man die Extraktivdestillation unter Verwendung
— einer Destillatabzugzone (= Trennzone C),
— einer Zulaufzone für das Ausgangs-$C_4$- oder $C_5$-Kohlenwasserstoffgemisch (= Trennzone A),
— einer Produktabzugzone für den Produktstrom (= Trennzone B) und
— einer Austreifzone, in der das selektive Lösungsmittel zurückgewonnen und aus der der die leichter löslichen Kohlenwasserstoffe enthaltende Strom abgezogen wird (= Trennzone D) durchführt, wobei man
a) das Ausgangs-$C_4$- oder $C_5$-Kohlenwasserstoffgemisch der Trennzone A zuführt,
b) das der Extraktivdestillation zuzuführende selektive Lösungsmittel im oberen Teil der Trennzone C zugibt,
c) das im unteren Teil der Trennzone C erhaltene beladene selektive Lösungsmittel zu einem Teil im oberen Teil der Trennzone A und zum anderen Teil im oberen Teil der Trennzone B zuführt,
d) das im unteren Teil der Trennzone A erhaltene beladene selektive Lösungsmittel und das im unteren Teil der Trennzone B erhaltene beladene selektive Lösungsmittel der Trennzone D zuführt, aus der der die leichter löslichen Kohlenwasserstoffe enthaltende Strom und ein Strom des von den Kohlenwasserstoffen ganz oder teilweise befreiten selektiven Lösungsmittels abgezogen werden,
e) aus der Trennzone B einen das konjugierte Diolefin und/oder Olefin bzw. Olefingemisch enthaltenden Produktstrom abzieht und
f) aus der Trennzone C das Destillat abzieht.

2. Verfahren nach Anspruch 1, dadurch gekennzeichnet, dass die im Gegenstrom zum selektiven Lösungsmittel in den parallelen Trennzonen A und B aufsteigenden Kohlenwasserstoffe ganz oder teilweise dem unteren Teil der Trennzone C zugeführt werden.

3. Verfahren gemäss Anspruch 1 und 2, dadurch gekennzeichnet, dass es auf die Gewinnung von 1,3-Butadien aus einem 1,3-Butadien enthaltenden $C_4$-Kohlenwasserstoffgemisch angewendet wird, welches gesättigte und einfach olefinisch ungesättigte $C_4$-Kohlenwasserstoffe als im

selektiven Lösungsmittel schwerer lösliche Kohlenwasserstoffe und höhere Acetylene und gegebenenfalls 1,2-Butadien als im selektiven Lösungsmittel leichter lösliche Kohlenwasserstoffe enthält.

4. Verfahren gemäss Anspruch 1 und 2, dadurch gekennzeichnet, dass es auf die Gewinnung von n-Buten oder n-Butengemischen aus einem n-Buten oder n-Buteneenthaltenden $C_4$-Kohlenwasserstoffgemisch angewendet wird.

5. Verfahren gemäss Anspruch 1 und 2, dadurch gekennzeichnet, dass es auf die Gewinnung von Isopren aus einem Isopren enthaltenden $C_5$-Kohlenwasserstoffgemisch angewendet wird.

6. Verfahren nach Anspruch 3, dadurch gekennzeichnet, dass man den 1,3-Butadien enthaltenden Produktstrom zur Feinreinigung einer destillativ wirkenden Trennzone (= Trennzone E) zuführt, der gereinigtes 1,3-Butadien als Kopf- oder als Seitenabzug entnommen wird, und das Sumpfprodukt der Trennzone E den Trennzonen A, B und/oder D zuführt.

7. Verfahren nach Anspruch 6, dadurch gekennzeichnet, dass die destillative Trennzone E zur Feinreinigung des 1,3-Butadiens zwei teilweise parallele Trennzonen enthält.

8. Verfahren nach Anspruch 1 bis 7, dadurch gekennzeichnet, dass zur gleichzeitigen Gewinnung eines weiteren Produktstromes eine weitere extraktivdestillativ wirkende, teilweise zu den Trennzonen A und B parallele Trennzone (= Trennzone G) angewendet wird.

9. Verfahren nach Anspruch 3 und 6, dadurch gekennzeichnet, dass man neben dem 1,3-Butadien enthaltenden Produktstrom einen n-Buten oder n-Butengemisch enthaltenden Produktstrom gewinnt.

10. Verfahren nach Anspruch 1 bis 9, dadurch gekennzeichnet, dass neben dem Ausgangs-$C_4$- oder $C_5$-Kohlenwasserstoffgemisch den Trennzonen A, B, C und/oder D ein weiterer bzw. weitere aufzutrennende $C_4$ oder $C_5$-Kohlenwasserstoffgemische, die sich in der Zusammensetzung vom Ausgangs-$C_4$- oder $C_5$-Kohlenwasserstoffgemisch unterscheiden, zugeführt werden.

11. Verfahren nach Anspruch 1 bis 10, dadurch gekennzeichnet, dass neben dem aus Trennzone B abgezogenen Produktstrom aus den Trennzonen A, B, C und/oder D ein weiterer bzw. weitere Produktströme abgezogen werden.

12. Verfahren nach Anspruch 1 bis 11, dadurch gekennzeichnet, dass zwei oder mehr als zwei parallele Trennzonen in einem Kolonnenmantel untergebracht werden.

13. Verfahren nach Anspruch 12, dadurch gekennzeichnet, dass die parallelen Trennzonen durch senkrechte Trennwände oder konzentrische Rohre voneinander getrennt werden.

**Claims**

1. A process for recovering a conjugated diolefin and/or olefin or olefin mixture from a $C_4$- or $C_5$-hydrocarbon mixture which contains the conjugated diolefin and/or the olefin or olefins, hydrocarbons which are more sparingly soluble in the selective solvent than the said diolefin and/or the olefin or olefins, and hydrocarbons which are more readily soluble in the said solvent than the said diolefin and/or the olefin or olefins, the $C_4$- or $C_5$-hydrocarbon mixture being separated, by means of extractive distillation, into a distillate containing the more sparingly soluble hydrocarbons, a product stream containing the conjugated diolefin and/or the olefin or olefin mixture, and a stream containing the more readily soluble hydrocarbons, wherein the extractive distillation is carried out using

— a distillate take-off zone (separation zone C),
— a feed zone for the starting $C_4$- or $C_5$-hydrocarbon mixture (separation zone A),
— a product take-off zone for the product stream (separation zone B) and
— a stripping zone in which the selective solvent is recovered and from which the stream containing the more readily soluble hydrocarbons is taken off (separation zone D),
and

(a) the starting $C_4$- or $C_5$-hydrocarbon mixture is fed to separation zone A,

(b) the selective solvent to be fed to the extractive distillation is introduced into the upper part of separation zone C,

(c) some of the laden selective solvent obtained in the lower part of separation zone C is fed to the upper part of separation zone A and the remainder is fed to the upper part of separation zone B,

(d) the laden selective solvent obtained in the lower part of separation zone A, and that obtained in the lower part of separation zone B, are fed to separation zone D, from which the stream containing the more readily soluble hydrocarbons, and a stream of the selective solvent which has been partially or completely freed from the hydrocarbons, are removed,

(e) a product stream containing the conjugated diolefin and/or the olefin or olefin mixture is taken off from separation zone B, and

(f) the distillate is removed from separation zone C.

2. A process as claimed in claim 1, wherein some or all of the hydrocarbons rising countercurrent to the selective solvent in the parallel separation zones A and B are fed to the lower part of separation zone C.

3. A process as claimed in claims 1 and 2, which is used for recovering buta-1,3-diene from a buta-1,3-diene-containing $C_4$-hydrocarbon mixture which contains saturated and monoolefinically unsaturated $C_4$-hydrocarbons as hydrocarbons which are more sparingly soluble in the selective solvent, and higher acetylenes, with or without buta-1,2-diene, as hydrocarbons which are more readily soluble in the selective solvent.

4. A process as claimed in claims 1 and 2, which is used for recovering n-butene or an n-butene mixture from a $C_4$-hydrocarbon mixture containing n-butene or n-butenes.

5. A process as claimed in claims 1 and 2, which is used for recovering isoprene from an isoprene-containing $C_5$-hydrocarbon mixture.

6. A process as claimed in claim 3, wherein the product stream containing buta-1,3-diene is fed to a distillative separation zone (separation zone E) to effect a high degree of purification, the purified buta-1,3-diene is removed as the top product or sidestream, and the bottom product of separation zone E is fed to separation zones A, B and/or D.

7. A process as claimed in claim 6, wherein, in order to purify buta-1,3-diene to a high degree, the distillative separation zone E contains two separation zones which run parallel for part of their lengths.

8. A process as claimed in claims 1 to 7, wherein, to effect simultaneous recovery of a further product stream, another separation zone (separation zone G) in which extractive distillation takes place is employed, this zone running parallel to separation zones A and B for part of its length.

9. A process as claimed in claims 3 and 6, wherein, in addition to the buta-1,3-diene-containing product stream, a product stream containing n-butene or an n-butene mixture is recovered.

10. A process as claimed in claims 1 to 9, wherein, in addition to the starting $C_4$- or $C_5$-hydrocarbon mixture, a further $C_4$- or $C_5$-hydrocarbon mixture to be separated, or further such mixtures, which differ in composition from the starting $C_4$- or $C_5$-hydrocarbon mixture, are fed to separation zones A, B, C and/or D.

11. A process as claimed in claims 1 to 10, wherein a further product stream or further product streams are taken off from separation zones A, B, C and/or D, in addition to the product stream removed from separation zone B.

12. A process as claimed in claims 1 to 11, wherein two or more parallel separation zones are located in one column jacket.

13. A process as claimed in claim 12, wherein the parallel separation zones are separated from one another by vertical partitions or concentric tubes.

## Revendications

1. Procédé pour l'obtention d'une dioléfine conjuguée et/ou d'une oléfine ou d'un mélange d'oléfines à partir d'un mélange d'hydrocarbures en $C_4$ ou $C_5$ contenant la dioléfine conjuguée et/ou l'oléfine ou les oléfines, mélange qui contient des hydrocarbures plus difficilement solubles dans le solvant sélectif que la dioléfine conjuguée et/ou l'oléfine ou les oléfines et des hydrocarbures plus facilement solubles dans le solvant sélectif que la dioléfine conjuguée et/ou l'oléfine ou les oléfines, procédé dans lequel le mélange d'hydrocarbures en $C_4$ ou $C_5$ est séparé, par distillation extractive, en un distillat contenant les hydrocarbures plus difficilement solubles, un courant de produit contenant la dioléfine conjuguée et/ou l'oléfine ou le mélange d'oléfines et un courant contenant les hydrocarbures plus facilement solubles, caractérisé en ce que l'on effectue la distillation extractive avec utilisation
— d'une zone d'extraction du distillat (= zone de séparation C),
— d'une zone d'introduction pour le mélange d'hydrocarbures en $C_4$ ou $C_5$ de départ (= zone de séparation A),
— d'une zone d'extraction du produit pour le courant de produit (= zone de séparation B), et
— d'une zone d'extraction dans laquelle le solvant sélectif est récupéré et à partir de laquelle le courant contenant les hydrocarbures plus facilement solubles est extrait (= zone de séparation D), et, dans ces conditions,
a) on introduit le mélange d'hydrocarbures en $C_4$ ou $C_5$ de départ dans la zone de séparation A,
b) on ajoute le solvant sélectif à envoyer vers la distillation extractive dans la partie supérieure de la zone de séparation C,
c) on dirige le solvant sélectif chargé, obtenu dans la partie inférieure de la zone de séparation C, pour une part vers la partie supérieure de la zone de séparation A et pour le reste vers la partie supérieure de la zone de séparation B,
d) on dirige le solvant sélectif chargé, obtenu dans la partie inférieure de la zone de séparation A vers la zone de séparation D, à partir de laquelle le courant contenant les hydrocarbures plus facilement solubles et un courant du solvant sélectif, complètement ou partiellement débarrassé des hydrocarbures, sont extraits,
e) on extrait de la zone de séparation B un courant de produit contenant la dioléfine conjuguée et/ou l'oléfine ou le mélange d'oléfines, et
f) on extrait le distillat de la zone de séparation C.

2. Procédé selon la revendication 1, caractérisé en ce que les hydrocarbures qui remontent dans les zones de séparation A et B parallèles, à contre-courant du solvant sélectif, sont dirigés en totalité ou en partie vers la partie inférieure de la zone de séparation C.

3. Procédé selon la revendication 1 ou 2, caractérisé en ce qu'il est appliqué à l'obtention de 1,3-butadiène à partir d'un mélange d'hydrocarbures en $C_4$ contenant du 1,3-butadiène, mélange qui contient des hydrocarbures en $C_4$ saturés et une fois insaturés oléfiniquement n tant qu'hydrocarbures plus difficilement solubles dans le solvant sélectif, ainsi que des hydrocarbures acétyléniques supérieurs et, le cas échéant, du 1,2-butadiène en tant qu'hydrocabures plus facilement solubles dans le solvant sélectif.

4. Procédé selon la revendication 1 ou 2, caractérisé en ce qu'il est appliqué à l'obtention de n-butène ou de mélanges de n-butènes à partir d'un mélange d'hydrocarbures en $C_4$ contenant du n-butène ou des n-butènes.

5. Procédé selon la revendication 1 ou 2, caractérisé en ce qu'il est appliqué à l'obtention d'isoprène à partir d'un mélange d'hydrocarbures en $C_5$ contenant de l'isoprène.

6. Procédé selon la revendication 3, caractérisé en ce qu'on dirige le courant de produit contenant du 1,3-butadiène, en vue de la purification poussée, vers une zone de séparation à action distillatoire (= zone de séparation E), d'où du 1,3-butadiène purifié est prélevé en tant qu'extrait de tête ou en tant qu'extrait latéral, et on dirige le produit de bas de colonne de la zone de séparation E vers les zones de séparation A, B et/ou D.

7. Procédé selon la revendication 6, caractérisé en ce que la zone de séparation distillatoire E pour la purification poussée du 1,3-butadiène comprend deux zones de séparation en partie parallèles.

8. Procédé selon l'une quelconque des revendications 1 à 7, caractérisé en ce que, pour l'obtention simultanée d'un autre courant de produit, on utilise une autre zone de séparation à action de distillation extractive, en partie parallèle aux zones de séparation A et B (= zone de séparation G).

9. Procédé selon la revendication 3 ou 6, caractérisé en ce qu'outre le courant de produit contenant du 1,3-butadiène, on obtient un courant de produit contenant du n-butène ou un mélange de n-butènes.

10. Procédé selon l'une quelconque des revendications 1 à 9, caractérisé en ce qu'outre le mélange d'hydrocarbures en $C_4$ ou $C_5$ de départ, on dirige vers les zones de séparation A, B, C et/ou D un autre ou d'autres mélanges d'hydrocarbures en $C_4$ ou $C_5$ à séparer, qui se différencient dans leur composition du mélange d'hydrocarbures en $C_4$ ou $C_5$ de départ.

11. Procédé selon l'une quelconque des revendications 1 à 10, caractérisé en ce qu'outre le courant de produit extrait de la zone de séparation B, un autre ou d'autres courants de produit sont extraits des zones de séparation A, B, C et/ou D.

12. Procédé selon l'une quelconque des revendications 1 à 11, caractérisé en ce que deux ou plus de deux zones de séparation parallèles sont logées dans une enveloppe de colonne.

13. Procédé selon la revendication 12, caractérisé en ce que les zones de séparation parallèles sont séparées les unes des autres par des cloisons verticales ou des tubes concentriques.

FIG.1

FIG. 2

FIG. 3

# FIG. 4

# FIG.5a

# FIG.5b

# FIG. 6a

# FIG. 6b